# EUROPEAN PATENT SPECIFICATION

(11) **EP 1 944 053 B1**
(45) Date of publication and mention of the grant of the patent: **21.10.2009**
(21) Application number: 07254723.5
(22) Date of filing: 06.12.2007
(51) Int. Cl.: A61M 25/09

(54) **A medical guide wire**
Medizinischer Führungsdraht
Guide-fil médical

(30) Priority: 28.12.2006 JP 2006355145
(43) Date of publication of application: 16.07.2008
(73) Proprietor: ASAHI INTECC CO., LTD., Nagoya-shi, Aichi-ken (JP)
(72) Inventor: Miyata, Naohiko, Nagoya-shi, Aichi-ken (JP); Nishiuchi, Makoto, Nagoya-shi, Aichi-ken (JP); Sugimura, Hiroo, Nagoya-shi, Aichi-ken (JP)
(74) Representative: Nicholls, Michael John

(56) References cited:
- EP-A- 0 806 220
- EP-A- 1 243 283
- EP-A- 1 388 348
- EP-A- 1 543 857
- US-A1- 2005 027 212
- US-B1- 6 645 159

## Description

The invention relates to a medical guide wire used upon inserting a medical device or equipment (e. g. , catheter) into a somatic cavity.

Various types of wires have been used to introduce a catheter for the purpose of vascular examination and treatment so that the guide wires are inserted into a target area of a somatic cavity prior to inserting the catheter.

Among the guide wires, one guide wire has a core line which is tapered off to form a cone-shaped configuration, and having a sheath body which is placed around a distal end portion of the core line. Some guide wires have a synthetic resin film coated on an outer surface of the core line.

In order to smoothly advance the guide wire through an arcuate area of the somatic cavity upon introducing the catheter, physical properties required for the guide wire are a good torque-transmission and supportability to hold the catheter and other related equipments inserted into the somatic cavity along the guide wire. Also required for a distal end portion of the guide wire is a good restitution capable to return the guide wire to an original configuration from a curved configuration.

To the distal end portion of the core line, used is a superelastic alloy wire (e. g., Ni-Ti based alloy wire) which has a low rigidity, and a stainless steel wire is used to a proximal end portion of the core line, in an attempt to enable an operator to a good torque-transmission and supportability at the proximal end portion while insuring a good restitution at the distal end portion of the core line, as disclosed by Japanese Laid-open Patent Application No. 2004-230141.

The prior guide wire of the above reference, however, employs a welding or soldering procedure to connect the two different wires at their butted ends. In order to insure a sufficient strength at the butted ends of the core line, it is necessary for the core line to have a certain amount of diameter (thickness), so that the butted ends are usually located at 200-300 mm on and proximally from the distal end of the core line. This renders the supportability of the guide wire insufficient at 100-200 mm from the distal end of the core line upon holding the catheter and other related equipments inserted into the somatic cavity along the guide wire.

EP 1 543 857 discloses a guide wire constructed of a first wire and a second wire that are connected to each other by welding at a joint that may be convex.

The welding and soldering procedures deteriorate the torque-transmission more as the butted ends position remote from the distal 1 end of the core line. Additionally, these procedures result in the butted ends being heat treated, so as to make the butted ends soften to reduce its physical, strength.

Therefore, the present invention is made to overcome the above drawbacks, and it is an object of the invention to provide a medical guide wire which is capable of bonding a proximal side wire and a rigidity-reduced distal side wire at a more diameter-decreased portion of the core line with a sufficient physical strength, so as to attain a good balance of torque-transmission, supportability and restitution at more distal end portion without losing a good torque-transmission and supportability even when bonded more in the proximity of the distal end portion.

According to the invention, there is provided a medical guide wire having a core line, a distal end portion of which is tapered off so that the distal end portion diametrically decreases progressively as approaching forward. A flexible sheath body is placed at least around the distal end portion of the core line. The core line has a proximal side wire and a distal side wire, the latter of which is to be bonded to a front end of the proximal side wire, the distal side wire being smaller in rigidity and superior in restitution than the proximal side wire. A concave portion is provided on either the distal end surface of the proximal side wire or a proximal end surface of the distal side wire. A convex portion is provided on the other side wire among the distal end surface of the proximal side wire and the proximal end surface of the distal side wire. The convex portion is interfit to the concave portion to be pressure bonded by means of swaging or drawing procedure.

Since the physical strength of the bonded portion between the proximal side wire and the distal side wire, depends on a frictional force due to the pressure-bonding procedure and the rupture strength of the bonded portion, it is possible to insure a sufficient strength at a diameter-decreased distal portion of the core line even when the proximal side wire and the distal side wire are bonded at more distally and more diameter-decreased portions of the core line. This enables the operator to achieve a good balance of torque-transmission, supportability and restitution at more distally end portion without losing a good torque-transmission and supportability even more in the proximity of the distal end portion of the core line.

Furthermore, according to the invention, a reduction ratio of cross sectional area of the concave portion and the convex portion is 40-60% when the distal side wire and the proximal side wire are subjected to the swaging or drawing procedure at the concave portion and the convex portion.

The reduction ratio this degree makes it possible to bond the two wires with a sufficient physical strength without inviting a disconnection.

Optionally, the distal side wire is composed of stranded wire elements.

The stranded wire elements permit the wire elements to relatively slide slightly each other to insure a positional freedom, thus making it possible to restrain strains to appear on an outer surface of the stranded wire elements, and insuring a good restitution from a curved configuration to an original configuration, compared to a distal side wire helically wound by a consecutive single wire element.

Optionally, the number of the stranded wire elements is 3-5.

Optionally, the stranded wire elements are stainless steel wires, superelastic alloy wires or combination of stainless steel wires and superelastic alloy wires.

Optionally, the flexible sheath body is a helical body placed around a distal end portion of the core line.

Optionally, the flexible sheath body is a synthetic resin film coated on an outer surface of the core line.
Fig. 1 is a side elevational view of a medical guide wire with a main portion partly sectioned according to a first embodiment of the invention;
Fig. 2 is a longitudinal cross sectional view of a main portion of the medical guide wire;
Fig. 3 is a latitudinal cross sectional view taken along line I-I of Fig. 2;
Fig. 4 is a latitudinal cross sectional view taken along line Ii-Ii of Fig. 2;
Fig. 5 is a longitudinal cross sectional view of a main portion of the medical guide wire according to a second embodiment of the invention;
Fig. 6 is a latitudinal cross sectional view taken along line V-V of Fig. 5;
Fig. 7 is a latitudinal cross sectional view taken along line Vi-Vi of Fig. 5;
Fig. 8 is a latitudinal cross sectional view in the proximity of a bonded portion between a distal side wire and a proximal side wire;
Fig. 9 is a latitudinal cross sectional view of a distal end portion of the distal side wire;
Fig. 10 is a perspective view of a single wire and stranded wires placed into a round pipe;
Fig. 11 is a graphical representation of a residual angle against the single wire and the stranded wires ;
Fig. 12 is a latitudinal cross sectional view of the stranded wires, outer surfaces of which are ground; and
Fig. 13 is a graphical representation between an outer diameter of the stranded wires and a maximum bending load.

In the following description of the depicted embodiments, the same reference numerals are used for features of the same type.

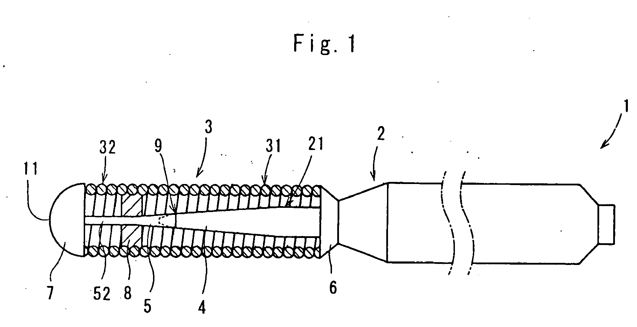

Referring to Figs. 1 through 4 which show a medical guide wire 1 (abbreviated merely as "guide wire 1" hereinafter) according to a first embodiment of the invention, the right hand side of the drawings is a proximal side, and the left hand side of the drawings is a distal side of the guide wire 1.

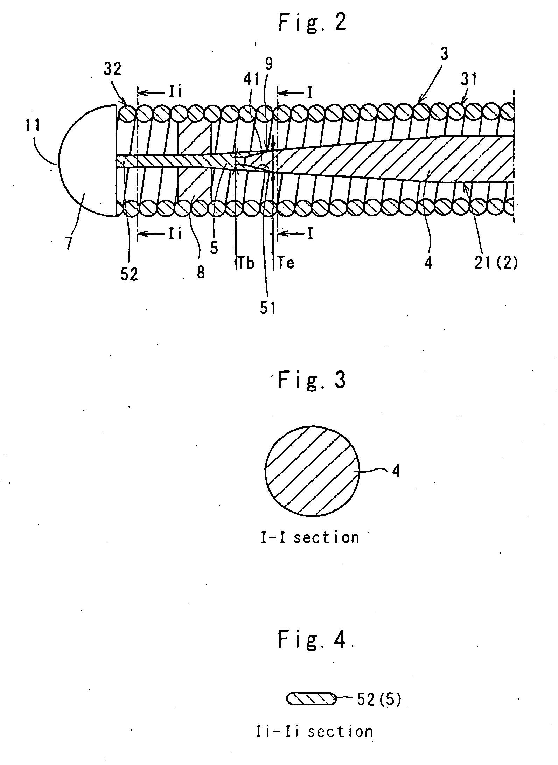

As shown in Figs. 1, 2, the guide wire 1 has a core line 2 and a helical body 3 placed around a distal end portion 21 of the core line 2 as a flexible sheath body.

The core line 2 has a distal end portion tapered off so that the distal end portion diametrically decreases progressively as approaching forward. The core line 2 has a proximal side wire 4 and a distal side wire 5, the latter of which is to be bonded to a distal end of the proximal side wire 4. The distal side wire 5 is smaller in rigidity and superior in restitution (anti-kink property) than the proximal side wire 4. The proximal side wire 4 is circular in cross section as shown in Fig. 3.

By way of illustration, the proximal side wire 4 is made of a stainless steel wire, and the distal side wire 5 is made of a superelastic wire (e.g., Ni-Ti based wire) which is smaller in rigidity and superior in restitution than the stainless steel wire. The distal side wire 5 and the proximal side wire 4 are bonded together as described in detail hereinafter.

The helical body 3 shaped into a coiled structure by a metallic material, is placed around an outer surface of the distal end portion 21 of the core line 2. The helical body 3 has a proximal end secured to the core line 2 by means of a soldering procedure and having a distal end secured to a plug head 7 together with a distal end of the core line 2. The plug head 7 is made of a soldering material. The helical body 3 also has a middle portion (near the distal end) secured to the core line 2 by means of a soldering material 8.

By way example, a proximal side of the helical body 3 acts as a radiotransparent helical line 31 and a distal side of the helical body 3 serves as a radiopaque helical line 32 with the soldering material 8 located at a boundary between the two helical lines 31, 32.

Upon bonding the distal side wire 5 and the proximal side wire 4 together, a concave portion 51 is provided on a proximal end surface of the distal side wire 5, and a convex portion 41 is provided on a distal end surface of the proximal side wire 4 in correspondence to the concave portion 51. The proximal side wire 4 interfits the convex portion 41 into the concave portion 51 of the distal side wire 5. Thereafter, the concave portion 51 is drawn through a pultrusion die (not shown) to stretch the concave portion 51 in its lengthwise direction, thus forcibly reducing a diameter of the concave portion 51 against the convex portion 41 so as to implement the pressure bonding between the distal side wire 5 and the proximal side wire 4.

In this instance, a reduction ratio of a cross sectional area of the concave portion 51 is preferably 40-60% when the concave portion 51 is drawn through the pultrusion die to lengthwisely stretch the concave portion 51. When the reduction ratio of the cross sectional area terminates short of the 40%, it becomes difficult to insure a required strength at a bonded portion 9 between the concave portion 51 and the convex portion 41. When the reduction ratio of the cross sectional area exceeds 60%, there may arise a disconnection at the core line 2 upon drawing the concave portion 51 through the pultrusion die. After the concave portion 51 is drawn, an outer surface of the concave portion 51 is ground to eliminate a diametrical difference at the bonded portion 9 between the distal side wire 5 and the proximal side wire 4.

The helical body 3 measures 200-300 mm in length, and a distance measures 60-100 mm between a distal end 11 of the guide wire 1 and the bonded portion 9. Flexibility is required for the guide wire 1 especially within the range of 60-100 mm from the distal end 11 of the guide wire 1 because the range is used when a medical device or equipment is introduced into the coronary and the aorta. The soldering material 8 is located at a boundary between the radiotransparent helical line 31 and the radiopaque helical line 32, which places the soldering material 8 remote by 25-35 mm from the distal end 11 of the guide wire 1. This means that the bonded portion 9 positions near the proximal side more than the boundary (i.e., soldering material 8) approaches near the proximal side.

The helical body 3 has an outer diameter of 0.25-0.4 mm, and the core line 2 has an outer diameter of 0.3-0.4 mm at the proximal end side which is remote from a distal end portion 21 (of the core line 2), around which the helical body 3 is placed.

Before the concave portion 51 is drawn through the pultrusion die, it is preferable that the distal side wire 5 has the distal end surface, an outer diameter of which measures 0.23-0.32 mm, and having the concave portion 51, an outer diameter of which measures 0.15-0.20 mm with the depth as 3.0-10.0 mm.

After the concave portion 51 is drawn, the concave portion 51 is tapered off at its inner surface so that the concave portion 51 preferably has a bottom portion Tb 4/10 times as diametrically large as an open entrance periphery Te of the concave portion 51. In correspondence to the inner shape of the concave portion 51, the convex portion 41 is formed into a cone-shaped configuration. The concave portion 51 thus tapered off prevents a stress-concentration and keep a continuity of rigidity at the bonded portion 9 when the convex portion 41 is bonded to the concave portion 51 for connection.

After the concave portion 51 is drawn, the bonded portion 9 measures 0.1-0.2 mm in outer diameter with the depth of the concave portion 51 as 5.0-15.0 mm. The distal side wire 5 has a distal end 52 ground until its outer diameter reduces to 0.045-0.065 mm, and pressed flat with 0.06-0.09 mm in breadth and 0.025-0.035 mm in thickness as shown in Fig. 4.

With the structure thus described, the distal side wire 5 and the proximal side wire 4 are pressure bonded by interfitting the convex portion 41 into the concave portion 51 to form the bonded portion 9.

The pressure-bonding procedure is such that it is possible to insure a sufficient strength at the bonded portion 9 even when the two side wires 4, 5 are bonded at a more distal end side of the core line 2 because the strength of the bonded portion 9 depends on the frictional force between the convex portion 41 and the concave portion 51.

In the guide wire 1 in which the reduction ratio of the cross sectional area of the concave portion 51 is 40-60%, the strength of the bonded portion 9 is far greater than the rupture strength (350-800 gf) of a distal end of the distal side wire 5, so as to produce a sufficinet strength at the bonded portion 9. It is to be noted that the bonded portion 9 substantially remain the rupture strength unchangeable regardless of the depth of the concave portion 51.

The pressure-bonding procedure accompanies no amount of heat which causes to reduce the material strength, as opposed to the brazing, soldering and welding procedures.

Since it is possible to insure a sufficient strength at a diameter-decreased distal portion of the core line 2 when the proximal side wire 4 and the distal side wire 5 are bonded at more distally and more diameter-decreased portions of the core line 2. This enables the operator to achieve a good balance of torque-transmission, supportability and restitution at a more distal end portion without losing a good torque-transmission and supportability even more in the proximity of the distal end portion of the core line 2.

Because of the sufficient strength insured at the bonded portion 9 even when the bonded portion 9 is placed in less than 100 mm from the distal end 11 of the guide wire 1, it becomes possible to ameliorate the supportability at a linear region 100-300 mm from the distal end 11 of the guide wire 1 while maintaining a good restitution of the distal end portion of the guide wire 1 with an improved toqrue-transmission. As opposed the present invention, the prior guide wire has been insufficient in supportability at the linear region 100-300 mm from the distal end of the prior guide wire.

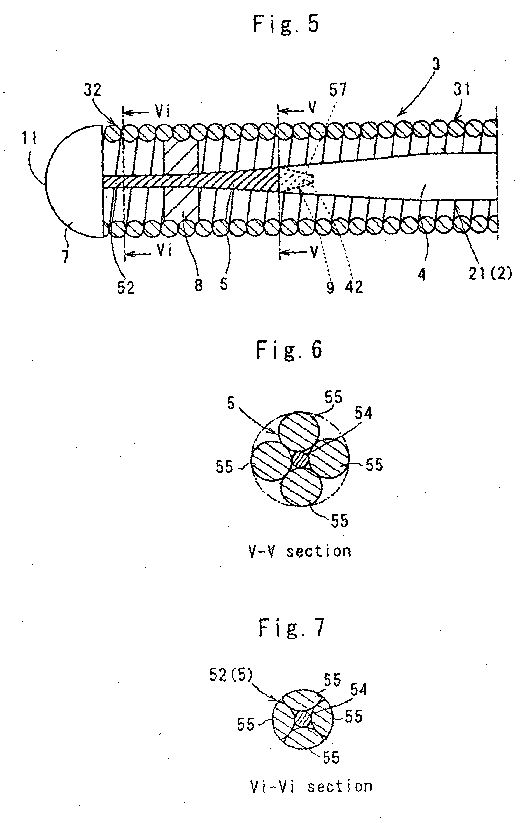

Figs. 5 through 13 show a second embodiment of the invention in which the right hand side indicates the proximal side, and the left hand side represents the distal side of the guide wire 1 in the drawings. Described in the second embodiment of the invention are component parts different from those of the first embodiment of the invention.

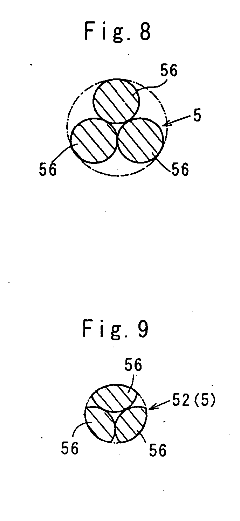

In the second embodiment of the invention, the distal side wire 5 of the guide wire 1 is stranded wires as shown in Figs. 5-7. Namely, the distal side wire 5 has five wire elements with four side wires 55 stranded around one thin core wire 54. The side wires 55 are thicker than the thin core wire 54 with both the wires 54, 55 made of high tensile strength stainless steel. Instead of the five wire elements, three wire elements 56 may be used as shown in Figs. 8, 9.

For the five wire elements, the thin core wire 54 measures 0.03-0.04 mm in outer diameter, and the side wire 55 measures 0.06-0.08 mm in outer diameter. For the three wire elements 56, each of the wire elements 56 measures 0. 08-0. 10 mm in outer diameter with an outer diameter of the stranded wires constituted preferably as 0. 15-0. 20 mm in terms of a good flexibility and restitution.

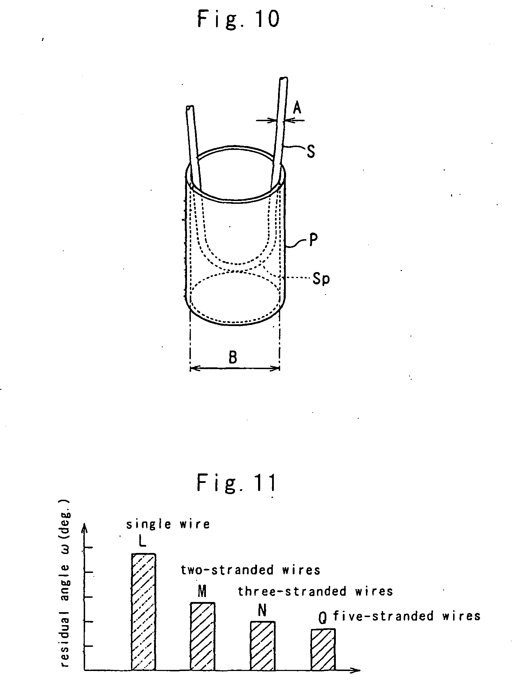

The reason why the structure of the stranded wires (consisting of 2-3 wire elements) is preferable, is explained below in reference to Figs. 10, 11.

Firstly, a single wire or stranded wires (each diameter: A) are prepared as specimens S. The curved portion Sp of the specimens S are pushed into a round pipe P (diameter: B = 3.0 mm) as shown in Fig. 10. After pulling the curved portion Sp out of the pipe P, the specimens S remains residual angles ( ω ) at the curved portion Sp, and measures the residual angles ( ω ) in Fig. 11.

In Fig. 11, a first specimen L is a single stainless steel wire with the outer diameter A as 0.19 mm. A second specimen M (two-stranded wires) is stranded wires consisting of two wire elements (each outer diameter: 0.1 mm) to represent its outer diameter A as 0. 19 mm. A third specimen N (three-stranded wires) is stranded wires which consist of three wire elements (each outer diameter: 0.09 mm) to represent its outer diameter A as 0. 19 mm. A fourth specimen Q (five-stranded wires) is stranded wires consisting of one core wire (outer diameter: 0.04 mm), around which four side wires are stranded (each outer diameter: 0.08 mm) to represent its outer diameter A as 0.19 mm. It is to be noted that the wire elements of the two-stranded wires M, the three-stranded wires N and the five-stranded wires Q are all of high tensile strength stainless steel.

As shown in Fig. 11, it is apparent that the residual angles ( ω ) of the three-stranded wires N and the five-stranded wires Q are less than half the residual angle ( ω ) of the single wire L, and those stranded wires N, Q are superior in restitution. It is, therefore, preferable that the stranded wires consist of more than three wire elements. The stranded wires may consist of five wire elements, and preferably within the five wire elements since the stranded wires render the wire elements thin when the number of the wire elements exceeds five.

Upon bonding the distal side wire 5 and the proximal side wire 4, a concave portion 42 is provided on the distal end surface of the proximal side wire 4, and a convex portion 57 is provided on the proximal end surface of the distal side wire 5 in correspondence to the concave portion 42. The distal side wire 5 interfits the convex portion 57 into the concave portion 42 of the proximal side wire 4.

Thereafter, the concave portion 42 is drawn through the pultrusion die to stretch the concave portion 42 in its lengthwise direction, thus forcibly reducing a diameter of the concave portion 42 against the convex portion 57 so as to pressure bond the distal side wire 5 to the proximal side wire 4.

After the concave portion 42 is drawn through the pultrusion die, the bonded portion 9 is ground to eliminate the diametrical difference at the bonded portion 9 between the distal side wire 5 and the proximal side wire 4.

As shown in Figs. 7, 9, the distal side wire 5 has a distal end portion 52 ground so that the distal end portion 52 decreases its diameter progressively as approaching forward. As a result, the distal side wire 5 has an outer diameter of 0. 18 mm in the proximity of the bonded portion 9, and having an outer diameter of 0.07-0.09 mm at the distal end portion 52 of the distal side wire 5.

In the second embodiment of the invention, the distal side wire 5 interfits the convex portion 57 into the concave portion 42 to implement the pressure bonding between the distal side wire 5 and the proximal side wire 4 in the same manner as mentioned in the first embodiment of the invention. The stranded wire elements permit the wire elements 55 to relatively slide slightly each other to insure a positional freedom, thus making it possible to restrain strains to appear on an outer surface of the stranded wire elements 55, and insuring a good restitution from a curved configuration to an original configuration, compared to the distal side wire helically wound by the single wire element.

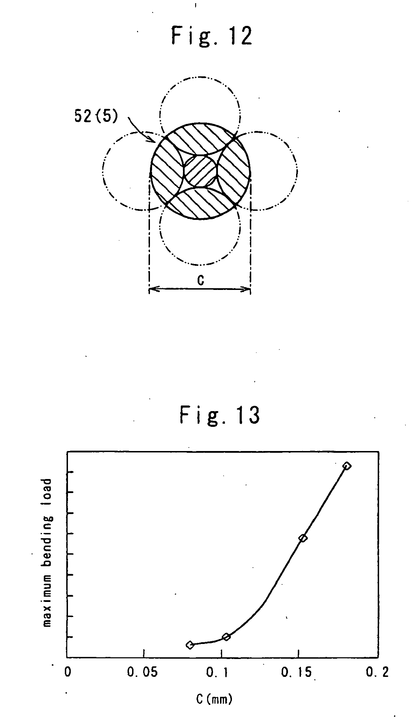

In reference to Figs. 12, 13, advantage derived by grinding the distal side wire 5 to have the outer diameter C of 0.07-0.09 mm at the distal end portion 52 of the distal side wire 5, is explained below.

The distal side wire 5 has the five-stranded wires Q (initial outer diameter: 0.18 mm) ground in several ways to have various diameters in order to observe a maximum bending load required to bend the five-stranded wires Q in a predetermined degree. The experimentation test is repeated with the result that the maximum bending load corresponds to the same flexible level as required for the distal end portion of the general guide wire when the distal side wire 5 has the outer diameter C of 0.07-0.09 mm at the distal end portion 52. The general guide wire deserves a guide wire formed by pressing a single stainless steel wire (outer diameter: 0.06 mm) into a flat wire (0.04 mm in thickness). With the distal end portion 52 ground to have the outer diameter C of 0.07-0.09 mm, it is possible to insure a sufficient flexibility and restitution for the distal end portion 52.

Although it is possible to insure the sufficient flexibility for the distal end portion 52 when the distal end portion 52 has the outer diameter C of less than 0.07 mm, it is preferable for the distal end portion 52 to have the outer diameter C of more than 0.07 mm when considering the physical strength required for the distal end portion 52.

### MODIFICATION FORMS

Instead of providing the concave portion 51 on the proximal end surface of the distal side wire 5, and the convex portion 41 on the distal end surface of the proximal side wire 4 in the first embodiment of the invention, the concave portion 51 may be on the proximal side wire 4, and the convex portion 41 may be on the distal side wire 5.

When the proximal side wire 4 makes its proximal side columnar immediately behind the bonded portion 9 of the proximal side wire 4, it is preferable to provide the concave portion 51 on the distal side wire 5, and the convex portion 41 is on the proximal side wire 4, in order to insure a smooth gradient transfer of rigidity from the bottom portion Tb to the open entrance periphery Te of the concave portion 51.

On the other hand, when the distal side wire 5 makes its distal side columnar immediately prior to the bonded portion 9 of the proximal side wire 4, it is preferable to provide the concave portion on the proximal side wire 4, and to provide the convex portion on the distal side wire 5.

When both the proximal side wire 4 and the distal side wire 5 make the respective distal and proximal sides continuously tapered off immediately behind and prior to the bonded portion 9, it is possible to insure a smooth gradient transfer of rigidity at the bonded portion 9 irrespective of whether the concave portion or the convex portion are provided on either the proximal side wire 4 or the distal side wire 5.

Instead of drawing the concave portion of the bonded portion 9 through the pultrusion die, a swaging procedure or a pressing procedure (with upper and lower die assembly) may be used to pressure bond the proximal side wire 4 and the distal side wire 5.

Instead of placing the bonded portion 9 proximally more than the boundary (soldering portion 8) between the radiopaque helical line 32 and the radiotransparent helical line 31, the bonded portion 9 may be distally placed more than the soldering portion 8.

It is to be appreciaed that the distal end portion 52 of the distal side wire 5 may be pressed flat in the second embodiment of the invention. In the first the embodiment of the invention, the distal side wire 5 is made of the superelastic alloy (e.g., Ni-Ti based alloy), the distal side wire 5 may be made of Ni-Ti-Co based alloy.

Instead of making the core wire 54 and the side wires 55 from the high tensile strength stainless steel in the second embodiment of the invention, all or some of the side wires 55 may be made of the superelastic alloy. Namely, the stranded wires may be wire elements made of the superelastic alloy or a combination of the stainless steel wire and the superelastic alloy wire. The three-stranded wire N may make all or some of the wire elements 56 from the superelastic alloy.

In the guide wire 1 in which the helical body 3 is placed around the core line 2 of the first and second embodiments of the invention, a synthetic resin film (not shown) may be coated as a flexible sheath body on an outer surface of the core line 2.

## Claims

1. A medical guide wire (1) in which a core line (2) is provided, a distal end portion of which is tapered off so that said distal end portion diametrically decreases progressively as approaching forward, and a flexible sheath body (3) is placed at least around said distal end portion of said core line (2),
wherein
said core line (2) having a proximal side wire (4) and a distal side wire (5), the latter of which is to be bonded to a front end of said proximal side wire (4), said distal side wire (5) being smaller in rigidity and superior in restitution than said proximal side wire (4);
a concave portion (42, 51) provided on either said distal end surface of said proximal side wire (4) or a proximal end surface of said distal side wire (5);
a convex portion (41, 57) provided on the other side wire among said distal end surface of said proximal side wire (4) and said proximal end surface of said distal side wire (5); and
said convex portion (41, 57) being interfit to said concave portion (42, 51) and pressure bonded by means of swaging or drawing procedure.
**characterised in that**
a reduction ratio of cross sectional area of said concave portion (42, 51) and said convex portion (41, 57) is 40-60% when said distal side wire (5) and said proximal side wire (4) are subjected to said swaging or said drawing procedure at said concave portion (42, 51) and said convex portion (41, 57) with said flexible sheath body (3) placed at least around said distal end portion of said core line (2).

2. The medical guide wire (1) according to claim 1, wherein said distal side wire (5) is composed of stranded wire elements (55, 56).

3. The medical guide wire (1) according to claim 2, wherein number of said stranded wire elements (55, 56) is 3-5 with said flexible sheath body (3) placed at least around said distal end portion of said core line (2).

4. The medical guide wire (1) according to claim 2, wherein said stranded wire elements (55, 56) are stainless steel wires, superelastic alloy wires or combination of stainless steel wires and superelastic alloy wires.

5. The medical guide wire (1) according to claim 3, wherein said stranded wire elements (55, 56) are stainless steel wires, superelastic alloy wires or combination of stainless steel wires and superelastic alloy wires.

6. The medical guide wire (1) according to claim 1, wherein said flexible sheath body (3) is a helical body placed around a distal end portion of said core line (2).

7. The medical guide wire (1) according to claim 2, wherein said flexible sheath body (3) is a helical body placed around a distal end portion of said core line (2).

8. The medical guide wire (1) according to claim 1, wherein said flexible sheath body (3) is a synthetic resin film coated on an outer surface of said core line (2).

9. The medical guide wire (1) according to claim 2, wherein said flexible sheath body (3) is a synthetic resin film coated on an outer surface of said core line (2).

## Patentansprüche

1. Medizinischer Führungsdraht (1), bei dem eine Kernleitung (2) vorgesehen ist, deren distaler Endabschnitt zulaufend abgetragen ist, so dass der distale Endabschnitt nach vorne hin zunehmend im Durchmesser abnimmt, und ein biegsamer Hüllenkörper (3) mindestens um den distalen Endabschnitt der Kernleitung (2) angeordnet ist,
wobei
die Kernleitung (2) einen Draht (4) auf der proximalen Seite und einen Draht (5) auf der distalen Seite aufweist, wobei letzerer mit einem vorderen Ende des Drahts (4) auf der proximalen Seite zu verbinden ist, wobei der Draht (5) auf der distalen Seite eine geringere Steifigkeit und eine größere Wiederherstellung aufweist als der Draht (4) auf der proximalen Seite;
ein konkaver Abschnitt (42, 51) entweder an der distalen Endfläche des Drahts (4) auf der proximalen Seite oder einer proximalen Endfläche des Drahts (5) auf der distalen Seite vorgesehen ist;
ein konvexer Abschnitt (41, 57) an dem Draht der anderen Seite zwischen der distalen Endfläche des Drahts (4) auf der proximalen Seite und der proximalen Endfläche des Drahts (5) auf der distalen Seite vorgesehen ist; und
der konvexe Abschnitt (41, 57) in den konkaven Abschnitt (42, 51) eingepasst und mittels eines Stauchhämmer- oder Ziehverfahrens druckverbunden ist,
**dadurch gekennzeichnet, dass**
ein Reduktionsverhältnis de Querschnittfläche des konkaven Abschnitts (42, 51) und des konvexen Abschnitts (41, 57) 40-60% beträgt, wenn der Draht (5) auf der distalen Seite und der Draht (4) auf der proximalen Seite an dem konkaven Abschnitt (42, 51) dem Stauchhämmer- oder dem Ziehverfahren unterzogen werden und der konvexe Abschnitt (41, 57) mit dem biegsamen Hüllenkörper (3) mindestens um den distalen Endabschnitt der Kernleitung (2) angeordnet ist.

2. Medizinischer Führungsdraht (1) nach Anspruch 1, **dadurch gekennzeichnet, dass** der Draht (5) auf der distalen Seite aus Litzendrahtelementen (55, 56) besteht.

3. Medizinischer Führungsdraht (1) nach Anspruch 2, **dadurch gekennzeichnet, dass** die Anzahl der Litzendrahtelemente (55, 56) 3 - 5 beträgt, wobei der biegsame Hüllenkörper (3) mindestens um den distalen Endabschnitt der Kernleitung (2) angeordnet ist.

4. Medizinischer Führungsdraht (1) nach Anspruch 2, **dadurch gekennzeichnet, dass** die Litzendrahtelemente (55, 56) Edelstahldrähte, superelastische Legierungsdrähte oder eine Kombination aus Edelstahldrähten und superelastischen Legierungsdrähten sind.

5. Medizinischer Führungsdraht (1) nach Anspruch 3, **dadurch gekennzeichnet, dass** die Litzendrahtelemente (55, 56) Edeistahldrähte, superelastische Legierungsdrähte oder eine Kombination aus Edelstahldrähten und superelastischen Legierungsdrähten sind.

6. Medizinischer Führungsdraht (1) nach Anspruch 1, **dadurch gekennzeichnet, dass** der biegsame Hüllenkörper (3) ein spiralförmiger Körper ist, der um einen distalen Endabschnitt der Kernleitung (2) angeordnet ist.

7. Medizinischer Führungsdraht (1) nach Anspruch 2, **dadurch gekennzeichnet, dass** der biegsame Hüllenkörper (3) ein spiralförmiger Körper ist, der um einen distalen Endabschnitt der Kernleitung (2) angeordnet ist.

8. Medizinischer Führungsdraht (1) nach Anspruch 1, **dadurch gekennzeichnet, dass** der biegsame Hüllenkörper (3) ein Kunstharzfilm ist, der auf eine Außenfläche der Kernleitung (2) aufgebracht ist.

9. Medizinischer Führungsdraht (1) nach Anspruch 2, **dadurch gekennzeichnet, dass** der biegsame Hüllenkörper (3) ein Kunstharzfilm ist, mit dem eine Außenfläche der Kernleitung (2) beschichtet ist.

## Revendications

1. Fil guide médical (1) dans lequel une ligne de noyau (2) est prévue, dont une partie d'extrémité distale rétrécit progressivement de sorte que ladite partie d'extrémité distale diminue diamétralement progressivement alors qu'elle s'approche de l'avant, et un corps d'enveloppe (3) flexible est placé au moins autour de ladite partie d'extrémité distale de ladite ligne de noyau (2),
dans lequel
ladite ligne de noyau (2) comporte un fil côté proximal (4) et un fil côté distal (5), ce dernier devant être lié à une extrémité avant dudit fil côté proximal (4), ledit fil côté distal (5) présentant une plus faible rigidité et une restitution plus élevée que ledit fil côté proximal (4) ;
une partie concave (42, 51) prévue soit sur ladite surface d'extrémité distale dudit fil côté proximal (4), soit sur une surface d'extrémité proximale dudit fil côté distal (5) ;
une partie convexe (41, 57) prévue sur le fil de l'autre côté parmi ladite surface d'extrémité distale dudit fil côté proximal (4) et ladite surface d'extrémité proximale dudit fil côté distal (5) ; et
ladite partie convexe (41, 57) étant assemblée à ladite partie concave (42, 51) et liée par pression au moyen d'une procédure d'estampage ou d'emboutissage,
**caractérisé en ce que**
un rapport de réduction des sections transversales de ladite partie concave (42, 51) et de ladite partie convexe (41, 57) est de 40 à 60 % lorsque ledit fil côté distal (5) et ledit fil côté proximal (4) sont soumis à ladite procédure d'estampage ou d'emboutissage au niveau de ladite partie concave (42, 51) et de ladite partie convexe (41, 57), ledit corps d'enveloppe (3) flexible étant placé au moins autour de ladite partie d'extrémité distale de ladite ligne de noyau (2).

2. Fil guide médical (1) selon la revendication 1, dans lequel ledit fil côté distal (5) est composé d'éléments de fil toronnés (55, 56).

3. Fil guide médical (1) selon la revendication 2, dans lequel le nombre desdits éléments de fil toronnés (55, 56) est de 3 à 5, ledit corps d'enveloppe (3) flexible étant placé au moins autour de ladite partie d'extrémité distale de ladite ligne de noyau (2).

4. Fil guide médical (1) selon la revendication 2, dans lequel lesdits éléments de fil toronnés (55, 56) sont des fils en acier inoxydable, des fils en alliage superélastique ou une combinaison de fils en acier inoxydable et de fils en alliage superélastique.

5. Fil guide médical (1) selon la revendication 3, dans lequel lesdits éléments de fil toronnés (55, 56) sont des fils en acier inoxydable, des fils en alliage superélastique ou une combinaison de fils en acier inoxydable et de fils en alliage superélastique.

6. Fil guide médical (1) selon la revendication 1, dans lequel ledit corps d'enveloppe (3) flexible est un corps hélicoïdal placé autour d'une partie d'extrémité distale de ladite ligne de noyau (2).

7. Fil guide médical (1) selon la revendication 2, dans lequel ledit corps d'enveloppe (3) flexible est un corps hélicoïdal placé autour d'une partie d'extrémité distale de ladite ligne de noyau (2).

8. Fil guide médical (1) selon la revendication 1, dans lequel ledit corps d'enveloppe (3) flexible est un film de résine synthétique déposé sur une surface externe de ladite ligne de noyau (2).

9. Fil guide médical (1) selon la revendication 2, dans lequel ledit corps d'enveloppe (3) flexible est un film de résine synthétique déposé sur une surface externe de ladite ligne de noyau (2).
